# EUROPEAN PATENT SPECIFICATION

(11) **EP 2 236 148 B1**
(45) Date of publication and mention of the grant of the patent: **16.08.2017**
(21) Application number: 08863646.9
(22) Date of filing: 17.12.2008
(51) Int. Cl.: A61K 36/15, A61P 31/04, A61P 1/00

(54) **CGNC FOR USE IN TREATING H. PYLORI INFECTION**
KONIFEREN-GRUENNADELKOMPLEX ZUR VERWENDUNG IN DER BEHANDLUNG VON H. PYLORI INFEKTION
COMPLEXE D'AIGUILLES VERTES DE CONIFÈRE POUR L'UTILISATION DANS LE TRAITEMENT DE L'INFECTION PAR H. PYLORI

(30) Priority: 26.12.2007 RU 2007149382
(43) Date of publication of application: 06.10.2010
(73) Proprietor: Roschin, Viktor Ivanovich, St.Petersburg 197220 (RU); SULTANOV, Vagif Sultanovich, St.Petersburg 195213 (RU)
(72) Inventor: ZHEBRUN, Anatolii Borisovich, St.Petersburg 190000 (RU); NIKITINA, Tamara Valentinovna, St.Petersburg 194223 (RU); SVARVAL, Alena Vladimirovna, St.Petersburg 197348 (RU)
(74) Representative: Ellmeyer, Wolfgang
(86) International application number: PCT/RU2008/000774
(87) International publication number: WO 2009/082267

(56) References cited:
- RU-C1- 2 021 803
- RU-C1- 2 264 822
- RU-C2- 2 208 950
- BESPALOV V G ET AL: "CONIFEROUS CHLOROPHYLL CAROTENE PASTEA REVIEW OF MEDICAL APPLICATIONS", N.N. Petrov State Scientific Research Institute of OncologyFederal Agency for Healthcare and Social Development of the Russian Federation , 2006, XP002671975, Retrieved from the Internet: URL:http://pineneedleresearch.com/media/CG NC_Dr_Bespalov_Review.pdf [retrieved on 2012-03-21]
- 'Khlorofillo-karotinovaya pasta (khvoinaya).' KHLOROFILLO-KAROTINOVAYA PASTA (KHVOINAYA)., [Online] 2004, XP008141349 Retrieved from the Internet: <URL:http://www.sumtech. ru/bank/medicine/archive/pasta.htm> [retrieved on 2008-11-19]

## Description

### Technological field

The invention belongs to the area of medicine and can be used as a therapeutic substance and as an inhibitor of the growth of *Helicobacter pylori* during anti-helicobacter therapy.

### Level of technological invention

*Helicobacter pylori (H. pylon)* is a well-researched human pathogen, and consists of a diverse number of bacterial strains which damage the gastrointestinal tract of mammals. *H. pylori* infection has a global reach and it is widespread. More than 60% of the entire population of the planet is infected with this bacterium. In Russia, the incidence rate of *H. pylori* infection is high. In Khakasia, Novosibirsk, and St-Petersburg, 80 to 95% of the population is infected.

The distinctive pattern of the epidemiological process of *H. pylori* in Russia results in the significant spreading of the infection among the adult population, lack of decline of *H. pylori* prevalence in general and spread among children and teenagers of diseases related to this microorganism, such as gastritis and ulcers (Zhebrun A.B., Helicobacter pylori infection, 2006, pp.1-379).

Histological and bacteriological data obtained from research performed on biopsies demonstrated that the use of anti-infective therapy on gastritis and related diseases lead to the disappearance of *H. pylori* from the mucosa (Rauws E., Tytgat G. Campylobacter pylori, Amsterdam, 1989, 169 pages). The Maastricht Consensus states that *H. Pylori* infection is the greatest risk factor for development of non-cardiac stomach cancer, which on average occurs in 71% of cases associated with *H. pylori.* Eradication of *H. pylori* prevents the development of precancerous changes in the mucosa of the stomach and is economically effective.

The use of anti-helicobacter therapy in the treatment of gastritis stops the progression of atrophic and metaplastic changes in mucosa. In some patients this treatment can lead to reduction of atrophy and possibly the reduction of metaplasia. In 2005, standards for "Diagnostics and therapy of acid-dependent diseases, including those associated with *H. pylon"* were approved (3rd Moscow Convention, 4 February 2005, Experimental and clinical gastroenterology, 2005, No.3 Appendix pp 1-4).

The quick development of resistance to antibiotics by new strains of *H. pylori* require flexible approaches to treatment to eradicate this bacterium and the development of first and second line and rescue therapy defence strategies.

Absolute indications for anti-helicobacter therapy are stomach and duodenal ulcer (in acute and remission phase), aggravated ulcer; mucosa-associated lyphoid tissue (MALT) lymphoma; atrophic gastritis; and conditions that occur after resection of the stomach due to cancer.

The Maastricht Consensus guidelines suggest that first line treatment should consist of triple therapy with a proton pump inhibitor, clarithromycin (in case of primary resistance to clarithromycin in corresponding region or population that does not exceed 15-20%), and amoxicillin or metronidazole for 7 days. Metronidazole should be considered in cases where resistance to this drug is less that 40%.

In cases where there is initial treatment failure due to the presence of antibiotic-resistant bacteria, quadruple therapy that includes drugs containing bismuth is recommended. In patients where both these treatment regimes fail, rescue therapy, based on antimicrobial susceptibility is recommended. Ineffective therapy results in bacterial damage to the stomach mucosa, and allows colonisation of the submucous area of the stomach. In this region there is a significantly higher pH and favourable conditions for the growth and reproduction of bacteria. Moreover, the bacteria have an increased tolerance to acidic conditions, and its survival in the stomach is dependent on the production of the enzymes urease and arginase. A urease test is a marker of this infection and a basis for detection of the infection in the body. The ability of the bacterium to survive in acidic conditions is one of the reasons why treatment is difficult.

The antibiotics metronidazole and amoxicillin, used in anti-helicobacter therapy, are powerful drugs that can cause adverse reactions. Furthermore, synthetic drugs can have toxic effects on the liver.

Metronidazole is an antibacterial and antiprotozoal substance used in the treatment of gastrointestinal tract diseases, lambliasis, amebiasis, and in combined therapy for stomach and duodenal ulcers.

Amoxicillin is a wide spectrum antibiotic that has an antibacterial and bactericidal effect and is active against most Gram-positive and Gram-negative bacteria. It is used for the treatment of bacterial infections in the urinary and gastrointestinal tract, and in combination therapy for stomach and duodenal ulcers.

The work of Baranskaya E.K. (Ulcer and *H. pylori* infection) can be considered the closest analogue to this invention. Journal "Digestive disorders" (2000, 2(1), pp. 8-14) describes anti-helicobacter therapy using antimicrobial drugs, and in particular, metronidazole. This antibiotic is used in all therapeutic regimes.

Shortcomings of traditional antibiotic therapy include adverse reactions involving the liver, blood and other organs. Moreover, resistance of *H. pylori* to these antibiotics results in the reduction of the effectiveness the treatment due to the appearance of new multi-resistant bacterial strains. Consequently, this leads to the inability of the treatment to fully suppress the growth of bacteria.

### Novelty of invention

The invention targets the development of a new therapeutic substance for *H. pylori* infections that will minimise adverse reactions and not contribute to the appearance of multi-resistant bacteria.

The present invention is defined by the claims.

This will be achieved as follows. The therapeutic substance for anti-helicobacter therapy is a Conifer Green Needle Complex (CGNC). The pharmaceutical composition for anti-helicobacter therapy contains a therapeutically effective amount of CGNC and pharmaceutically acceptable carriers and/or solvents. The pharmaceutical composition is prepared in the form of solution, suspension, capsule, or tablets.

Chlorophyll-carotene paste or Conifer Green Needle Complex (CGNC) is used as an active ingredient in the pharmaceutical composition for the anti-helicobacter therapeutical substance, Bioeffective A. It is an inhibitor of *H. pyLori* bacteria. The method of conducting anti-helicobacter therapy includes administration to patients an effective amount of CGNC in drug form or as a part of a pharmaceutical mixture.

CGNC is an extract from pine and spruce green needles. It is a thick, dark-green paste with a specific coniferous smell. It consists of 35-45% of water and extractive compounds. It is neutralised to pH - 7.8-9.0 by a water solution of caustic soda that has been extracted by hydrocarbon solvent. It has antimicrobial, wound healing, fungicidal, and interferonogenic activity.

The following table outlines the chemical composition of CGNC in % of weight of organic compounds.

**Table 1.**

| Component | Amount (% weight of organic compounds) |
|---|---|
| Chlorophyll derivatives | 1-2 |
| Beta-carotene and other carotenoids | 0.04-0.06 |
| Sodium salts of fatty, resin, dibasic, oxo- and oxyacids; including | 44-60 |
| pinifolic acid. | 10-25 |
| Waxes | 5-8 |
| Essential oils | 1-1.2 |
| Neutral compounds (alcohols, aldehydes, esters); including | 40-55 |
| unsaponifiable compounds; and | 20-30 |
| labdanoids. | 30-50 |

The invention is applied as follows.

Conducted investigations demonstrated a new property of CGNC was discovered. It was found that this substance inhibits the growth of *H. pylori* in an *in vitro* model. The effect of CGNC was compared with the antimicrobials metronidazole and amoxicillin.

The basis for the patent application of CGNC is the identification of previously unknown new activities for this compound.

Fibrogastroscopy was used to collect biopsy samples from 27 patients with disorders of the gastrointestinal tract (chronic gastritis, gastroduodenitis, atrophic gastritis, erosive gastritis, stomach and duodenal ulcer). Inoculation of biopsy material resulted in the isolation of 15 strains of *H. pylori.*

The effect of the following doses of CGNC was examined: 50, 100, and 300 mg/ml. Bacteriological methods were used to conduct the study. Three doses of CGNC (50, 100 and 300 mg/ml) were used for analysis of the CGNC effect on the growth of *H. pylori.* Serial dilutions, of dense nutrient media containing an increasing concentration of the test substance were set up. Isolates of *H. pylori* inoculated on standard for this type of bacteria mediums (without CGNC) were used to control the microbial growth. The effect of the test substance was compared with the effect of the placebo (sterile saline solution - 0.15 M NaCl, pH 7.0-7.2). In a comparative experiment, the effect of the antimicrobials, amoxicillin (20 µg/ml) and metronidazole (80 µg/ml), were evaluated.

The results obtained after the addition of CGNC, a substance of plant origin, to nutrient medium are presented in Table 2.

**Tables 2. Study of effect of CGNC on growth of Helicobacter pylori**

| Strain Nº | Number of passages | Results of growth of Helicobacter pylori, (days) | Concentration of test-substance (CGNC) in agar | | | | | | | | |
|---|---|---|---|---|---|---|---|---|---|---|---|
| | | | 50 (mg/ml) | | | 100 (mg/ml) | | | 300 (mg/ml) | | |
| | | | Urease test | Gram staining | Results | Urease test | Gram staining | Results | Urease test | Gram staining | Results |
| | 1st | 7 | | | | | | Growth of separate colonies | | | Inhibit growth |
| 700 | 2nd | 7, 12 | + | + | + | + | ± | | - | - | |
| | 3rd | 7 | | | | | | | | | |
| | 1st | 7 | | | | | | Growth of separate colonies | | | Inhibit growth |
| 707 | 2nd | 7, 12 | + | + | + | + | ± | | - | - | |
| | 3rd | 7 | | | | | | | | | |
| | 1st | 7 | | | | | | Growth of separate colonies | | | Inhibit growth |
| 737 | 2nd | 7,12 | + | + | + | + | ± | | - | - | |
| | 3rd | 7 | | | | | | | | | |
| | 1st | 7 | | | | | | Growth of separate colonies | | | Inhibit growth |
| 755 | 2nd | 7 | + | + | + | + | ± | | - | - | |
| | 3rd | 7 | | | | | | | | | |
| | 1st | 7, 12 | | | | | | Growth of separate colonies | | | Inhibit growth |
| 769 | 2nd | 7 | + | + | + | + | ± | | - | - | |
| | 3rd | 7 | | | | | | | | | |
| | 1st | 6 | | | | | | Growth of separate colonies | | | Inhibit growth |
| 773 | 2nd | 6, 12 | + | + | + | + | ± | | - | - | |
| | 3rd | 6 | | | | | | | | | |
| | 1st | 6 | | | | | | Growth of separate colonies | | | Inhibit growth |
| 774 | 2nd | 6 | + | + | + | + | ± | | - | - | |
| | 3rd | 6, 10 | | | | | | | | | |
| | 1st | 6 | | | | | | Growth of separate colonies | | | Inhibit growth |
| 781 | 2nd | 6 | + | + | + | + | ± | | - | - | |
| | 3rd | 6, 10 | | | | | | | | | |
| | 1st | 6 | | | | | | Growth of separate colonies | | | Inhibit growth |
| 782 | 2nd | 12 | + | + | + | + | ± | | - | - | |
| | 3rd | 6 | | | | | | | | | |
| | 1st | 6 | | | | | | Growth of separate colonies | | | Inhibit growth |
| 783 | 2nd | 7 | + | + | + | + | ± | | - | - | |
| | 3rd | 7, 12 | | | | | | | | | |
| | 1st | 6 | | | | | | Growth of separate colonies | | | Inhibit growth |
| 784 | 2nd | 6 | + | + | + | + | ± | | - | - | |
| | 3rd | 6, 12 | | | | | | | | | |
| | 1st | 6 | | | | | | Growth of separate colonies | | | Inhibit growth |
| 785 | 2nd | 6 | + | + | + | + | ± | | - | - | |
| | 3rd | 6, 10 | | | | | | | | | |
| | 1st | 6 | | | | | | Growth of separate colonies | | | Inhibit growth |
| 786 | 2nd | 6,12 | + | + | + | + | ± | | - | - | |
| | 3rd | 6 | | | | | | | | | |
| 787 | 1st | 6 | + | + | + | + | ± | Growth of separate colonies | - | - | Inhibit growth |
| | 2nd | 6, 10 | | | | | | | | | |
| 788 | 1st | 6 | | + | + | + | ± | Growth of separate colonies | | | Inhibit growth |
| | 2nd | 6 | + | | | | | | - | - | |
| | 3rd | 12 | | | | | | | | | |

### Legend:

Results of bacteriological analysis: (+) - growth of individual colonies
Urease test: (+) - positive
   (-)- negative
Gram staining: (+) - characteristic for H. pylori Gram staining
   (±) - presence of bacilliform and coccoid forms
   (-) - absence of microbe

CGNC, at a concentration of 300 mg/ml, inhibited the growth of all of the 15 strains of *H. pylori* that were tested. (maximum examination and observation period is 12 days).

At a concentration of 50 mg/ml, growth of *H*. *pylori was* not inhibited (maximum examination and observation period is 12 days). At 100 mg/ml CGNC, led to partial formation of coccoid forms of bacteria were observed. Microscopy of the bacterial colonies revealed the presence of a mixture of cocci and typically curved bacilli. Research has shown that *H. pylori* can exist in bacilli and cocci forms (typically under negative environmental growth conditions). The coccoid form of *H*. *pylori* can be found in bioptic samples of mucosa and under conditions of the artificial inoculation. The coccoid form of *H. pylori* cannot be cultured.

The transformation of *H.pylori* into a coccoid form suggests an adaptive characteristic that occurs for survival in unfavourable environmental conditions.

This suggests that CGNC at 100 mg/ml creates conditions that are unfavourable for the growth and reproduction of this microbe. Reversion of the bacterium to its vegetative form or restoration of its ability to reproduce was not recorded after passaging coccal form of *H.pylori.* It is considered that the coccal form of *H.pylori* does not belong to the phase of dormant viable bacteria, and represents a population of degenerative and dead cells.

Metronidazole and amoxicillin, the most common used therapeutical substances in combined anti-helicobacter therapy, were chosen as comparator-drugs. *H. pylori* isolated from patients (No. 782, 785, 786, 787), were used to compare the effect of CGNC (300 mg/ml), amoxicillin (20 µg/ml) and metronidazole (80 µg/ml) (Table 3). These strains were isolated from patients with chronic gastroduodenitis, gastritis associated with gastric stump, duodenal ulcer and reflux esophagitis. CGNC (300 mg/ml) and amoxicillin (20 µg/ml) inhibited the growth of these strains. Metronidazole (80 µg/ml) did not have an inhibiting effect on the growth of these strains (Table 3). The results comparing the effect of the test substance with the standard drugs used to treat *H. pylori* are presented in Table 3.

**Table 3. Study of the effect of comparator drugs on the growth of H. pylori**

| Strain number (patient code) | Diagnosis | Active substance | | | |
|---|---|---|---|---|---|
| | | Amoxicillin (20 µg/ml) | Metronidazole (80 µg/ml) | CGNC (300 mg/ml) | Placebo (saline solution pH = 7.0-7.2) |
| 782 | CGD | + | - | + | - |
| 785 | Gastritis associated with gastric stump | + | - | + | - |
| 786 | DU, reflux esophagitis | + | - | + | - |
| 787 | CGD | + | - | + | - |

### Legend:

CGD - chronic gastroduodenitis
DU - duodenal ulcer
(+) - inhibition of growth
(-) - no inhibition of growth

As the result of the conducted trials the following results were obtained:
At the concentration of 300 mg/ml CGNC had the inhibiting effect on the growth of Helicobacter pylori isolates (maximum examination and observation period is 12 days).

At 50 and 100 mg/ml CGNC did not inhibit the growth of *H. pylori* isolates (maximum examination and observation period is 12 days).

At the concentration of 100 mg/ml CGNC partially led to formation of coccal form of bacteria (microscopy of colonies revealed that part of bacteria was in coccal form and part in form of curved bacillus).

The placebo (sterile saline solution, pH 7.0-7.2), did not have any effect on the growth of the microorganism.

When the effect of CGNC (300 mg/ml) on the growth of *H. pylori* was compared with amoxicillin (20 µg/ml) and metronidazole (80 µg/ml), the results showed that inhibition of growth occurred with CGNC and amoxicillin. Metronidazole did not inhibit the growth of *H. pylori.*

Table 4 shows the results of an experiment to determine the minimal inhibitory concentration (MIC) of CGNC when media are inoculated with *H. pylori.* The MIC is the lowest concentration of the test substance that will inhibit the visible growth of the microorganism after overnight incubation.

**Table 4. Results of quantitative analysis of the inhibiting activity of CGNC on growth of H. pylori**

| Inoculating dose (No. *H. pylori* bacteria/dish) | CGNC concentration (mg/ml) | | | Control (pure culture of *H.pylori*) |
|---|---|---|---|---|
| | 50 | 100 | 300 | |
| 0.4x10⁹ | + | + | - | Continuous growth |
| 0.4x10⁸ | + | + | - | >20 colonies |
| 0.4x10⁷ | + | - | - | 20 colonies |
| 0.4x10⁶ | + | - | - | 10 colonies |
| 0.4x10⁵ | + | - | - | Up to 6 colonies |

### Legend:

+: growth of *H. pylori*
- : no growth

The results show that CGNC has a marked inhibiting effect on the growth of *H. pylori* (Table 4). The MIC of CGNC that causes complete inhibition of *H. pylori* (at a concentration of 0.4x10⁷ bacteria) is 100 mg/ml (Table 4).

The ability of CGNC to inhibit growth of *H. pylori* makes it suitable to be used as a therapeutic substance in combination therapy for disorders associated with this microorganism.

Although, when compared with amoxicillin, the required dose of CGNC for suppression of bacterial growth is higher, it has some advantages as a treatment. These are that:
- it is composed of natural compounds, leading to fewer side effects in humans than synthetic antibiotics or compounds;
- there is no "acquired tolerance" of pathogen's strains to this therapeutic substance, due to effect of a combination of compounds with bactericidal and bacteriostatic activity, such as: terpenes, resin acids, labdanoids, etc.;
- CGNC belongs to the Class IV Hazardous substances category, and this is favourably comparable with other substances used in helicobacter therapy;
- it is obtained readily from raw material (pine needles) that is considered waste by the logging industry;
- it can be used directly or in various therapeutic finished forms.

### Examples of preferred execution of the invention

Examples of pharmaceutical compositions include solid forms (tablet, pill, capsule, granules, etc.); liquid forms (solution, suspension, emulsion), therapeutic forms for oral administration, traditional forms for parenteral administration or as rectal suppositories.

The compositions are prepared using conventional methods. These methods include mixing active ingredients with a carrier (can include one or more excipients), and obtaining a finished product from the mixture.

Compositions for oral administration can contain traditional fillers and can be prepared in solid or liquid forms (tablet, capsule, solution, suspension or syrup). They can contain any acceptable excipients such as binding agents (sugar, gelatine, sorbitol, tragacanth, and polyvinylpyrrolidone), fillers (lactose, lecithin, starch, calcium phosphate, sorbitol), tableted lubricants (magnesium stearate), disintegrators (starch, polyvinylpyrrolidone, micro crystalline cellulose, and carboxymethyl cellulose), humidifiers (lauryl sulfate sodium), dispersing substances and surfactants. Liquid forms for oral administration can include solvents (water, vegetable or animal oils, and mineral oil), dextrose and other saccharide solutions, glycols, dispersing or surface active substances (SAS).

Tablets can be made by using a tablet press. An active ingredient, in powder or granule form is mixed (if necessary), with binding agents (for example, povidon, gelatine, or hydroxypropyl methylcellulose), lubricants, inert solvents, disintegrators (such as cellulose derivatives, crosslinked povidon, sodium carboxymethyl cellulose), or surface active or dispersing substances. Tablets made by moulding, using the corresponding equipment, contain a mixture of wetted powder of CGNC with inert liquid solvents. If required, tablets can be coated for slow or controlled release of an active ingredient. The coating can be made from hydroxypropyl starch, disodium phosphate or hydroxypropyl methylcellulose or with gelling agents (carrageenan, gelatine, waxes) at different ratios to obtain the preferred releasing profile.

Compositions for parenteral administration can be prepared by both traditional pharmaceutical methods (in a form of solutions, suspensions), and as water micro-emulsions (as per patent RU 2189231), based on Hank's solution and 10% ethanol. Other methods for preparation of CGNC for parenteral administration can involve including water, pharmaceutically acceptable fats or oils, alcohols or other organic solvents, surfactants and/or antioxidants, and preservatives. Typical concentrations of CGNC range from 0.05 to 80%. Finished compositions can contain a single dose or be produced as ampoules or vials that contain several single doses. If necessary, the finished therapeutic forms can contain stabilizers, buffer systems, and other excipients.

Agents used for the rectal suppository form can include substances such as paraffin, vegetable, animal or mineral fats or oils, emulsifiers, polyethylene glycol, lauryl sulfate or sulfate salts, mineral acids or sodium hydrocarbonate.

### Examples of pharmaceutical compositions:

### Example 1. Liquid peroral form.

| | |
|---|---|
| CGNC | 0.050 - 50 (w/w) |
| Purified and/or distilled | |
| and/or mineral water | up to 100 (w/w) |

CGNC is mixed with purified water at a specified ratio while heating to 40°C, stirred for 10-15 min and cooled to 5-8°C in a fridge. The mixture is allowed to stand at this temperature for 24 hours, filtered, sterilised in an autoclave, and dispensed into bottles with a dosing device.

### Example 2. Soft vegetable capsules

| | |
|---|---|
| CGNC | 22.7-33.2 (w/w) |
| Fillers: soy oil and/or lecithin, | |
| and/or silicon dioxide | 45.2-50.0 (w/w) |

Soft coating of vegetable capsules:

| | |
|---|---|
| Carrageenan | 4.9 (w/w) |
| Disodium phosphate | 0.4 (w/w) |
| Hydroxipropyl of starch | 15.1 (w/w) |
| Glycerine | 11.8 (w/w) |
| Purified water | up to 100 (w/w) |

The ingredients are mixed and placed in dark coloured capsules that contain the following natural ingredients: carrageenan, hydroxypropyl starch, glycerine, sodium phosphate and water.

The mixture is then packed into bottles or blisters, and sterilised.

### Example 3. Coated tablets

| | |
|---|---|
| CGNC | 15.38-49.23 (w/w) |
| Potato starch | 0.538 - 10.76 (w/w) |
| Lactose | 29.23 - 30.77 (w/w) |
| Microcrystalline cellulose with a water content of 5% | 18.0-40.0 (w/w) |
| Polyvinylpyrrolidone (PVP), medical, low molecular weight | 2.05-2.15 (w/w) |
| Calcium stearate | 0.92-0.94 (w/w) |

Tablets are produced in a "boiling layer" by mixing the main ingredients. The mixture is then wetted and a water solution of PVP is prepared separately and added to CGNC and stirred. The mixture is filtered through a fosta nylon sieve. After completion of supply of the wetting agent, the system is rinsed with warm water at 50-60°C and a pump is switched off. The mixture is dried at 55-60°C for 8-10 minutes and an apparatus is switched off and loaded with a specified amount of the dusting agents - microcrystalline cellulose and calcium stearate. The mixture is dried for 1-2 minutes at 55-60°C. After drying and dusting, the mixture is unloaded and sifted using a vibrating sieve. The finished granulate is compressed into 0.65 g tablets with a CGNC content of 0.1 -0.32 g, and packed into bottles or blisters.

### Industrial applications

As a result of the conducted investigations, it was established that CGNC has marked inhibiting effect on the growth of *H. pylori* in case of artificial cultivation of the bacteria on a dense nutritive medium.

The ability of CGNC to inhibit growth of *H. pylori* suggests that it can be used as a therapeutic substance in combination therapy for disorders associated with this microorganism.

## Claims

1. Composition comprising Conifer Green Needle Complex (CGNC) for use in the treatment of H.pylori infection.

2. Composition for use according to claim 1 wherein said composition contains pharmaceutically acceptable carriers and/or solvents.

3. Composition for use according to claim 1 or claim 2 wherein said composition is prepared in the form of a solution, suspension, capsule, tablet, granules or suppository.

4. Composition for use according to claim 3 wherein said treatment is inhibition of *H*. *pylori* growth.

## Patentansprüche

1. Zusammensetzung, umfassend den Komplex aus den grünen Nadeln von Koniferen ("Conifer Green Needle Complex", CGNC) zur Verwendung bei der Behandlung einer *H.-pylori-*Infektion.

2. Zusammensetzung zur Verwendung nach Anspruch 1, wobei die Zusammensetzung pharmazeutisch unbedenkliche Träger und/oder Lösungsmittel umfasst.

3. Zusammensetzung zur Verwendung nach Anspruch 1 oder 2, wobei die Zusammensetzung in Form einer Lösung, einer Suspension, einer Kapsel, einer Tablette, von Kügelchen oder von Zäpfchen hergestellt ist.

4. Zusammensetzung zur Verwendung nach Anspruch 3, wobei die Behandlung in der Hemmung von *H*.*-pylori*-Wachstum besteht.

## Revendications

1. Composition comprenant le complexe d'aguille verte de conifère (CGNC), destinée à être utilisé dans le traitement d'une infection par *H. pylori.*

2. Composition destinée à être utilisée selon la revendication 1, ladite composition contenant des excipients et/ou solvants.

3. Composition destinée à être utilisée selon les revendications 1 ou 2, ladite composition étant préparée sous forme de solution, suspension, capsule, cachet, granules ou suppositoire.

4. Composition destinée à être utilisée selon la revendication 3, ledit traitement étant l'inhibition de la croissance de *H. pylori.*
